# EUROPÄISCHE PATENTSCHRIFT

(11) **EP 0 449 777 B1**
(45) Veröffentlichungstag und Bekanntmachung des Hinweises auf die Patenterteilung: **10.05.1995**
(21) Anmeldenummer: 91810193.2
(22) Anmeldetag: 21.03.1991
(51) Int. Cl.: C07C 69/732, C07C 67/343

(54) **Verfahren zur Herstellung von Hydroxyphenylpropionaten**
Process for the preparation of hydroxyphenylpropionates
Procédé pour la préparation d'hydroxyphénylpropionates

(30) Priorität: 30.03.1990 CH 1057/90
(43) Veröffentlichungstag der Anmeldung: 02.10.1991
(73) Patentinhaber: CIBA-GEIGY AG, 4002 Basel (CH)
(72) Erfinder: Brogli, Franz, Dr., CH-4114 Hofstetten (CH); Kälin, Guido, CH-4414 Füllinsdorf (CH)

(56) Entgegenhaltungen:
- DE-A- 2 445 345
- DE-C- 3 390 557

## Beschreibung

Die vorliegende Erfindung betrifft ein neues Verfahren zur Herstellung von Hydroxyphenylpropionaten.

Hydroxyphenylpropionate werden meist durch sogenannte Michael-Addition aus einem Phenol und einem Acrylsäurealkylester in Gegenwart eines basischen Katalysators wie Kaliumhydroxid hergestellt. Auch wird beispielsweise in DE-C-33 90 557 die Verwendung von Kaliumphenolat und in JP-A-161 350/81 die Verwendung von Natrium- oder Kaliumphenolat als Katalysator beschrieben. Die Additionsprodukte, also Hydroxyphenylpropionate, können dann durch Kristallisation oder, wie in US-A-3,330,859 und US-A-3,364,250 ausgeführt, durch Destillation von Nebenprodukten befreit werden.

Die so erhaltenen Hydroxyphenylpropionate sind wichtige Zwischenprodukte für die Herstellung von Antioxidantien für synthetische Polymere, insbesondere Polyolefine, wie sie z.B. in den obigen Dokumenten beschrieben sind. Durch Verändern des Molekulargewichts der Estergruppe kann man bestimmten Anforderungen, die an ein Antioxidans gestellt werden, wie z.B. geringe Flüchtigkeit, gerecht werden. Dies ist direkt durch die Synthese, aber auch indirekt, beispielsweise durch Umesterung eines bereits hergestellten Hydroxyphenylpropionats, erreichbar.

Es hat sich nun gezeigt, dass die genannte Michael-Addition bedeutend vorteilhafter verläuft, wenn die Umsetzung von Phenol und Acrylsäurealkylester in Gegenwart eines Gemisches aus einem Natrium- und Kaliumphenolat erfolgt.

Gegenstand der vorliegenden Erfindung ist somit ein Verfahren zur Herstellung von Verbindungen der Formel 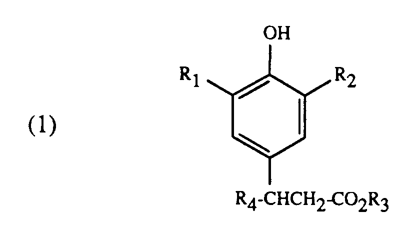
durch Umsetzung von Verbindungen der Formel

(2) R₄-CH=CH-CO₂R₃

mit Verbindungen der Formel 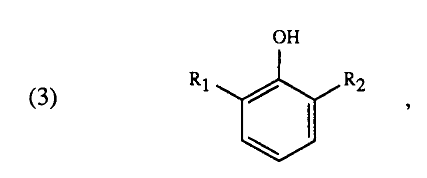
worin R₁ bis R₃ unabhängig voneinander Alkyl mit 1 bis 4 Kohlenstoffatomen sind, und R₄ Wasserstoff oder Alkyl mit 1 bis 4 Kohlenstoffatomen ist, in Gegenwart einer Base und Isolierung der Verbindungen der Formel (1), dadurch gekennzeichnet, dass die Umsetzung einer Verbindung der Formel (2) mit einer Verbindung der Formel (3) in Gegenwart eines Gemisches aus den Verbindungen der Formeln 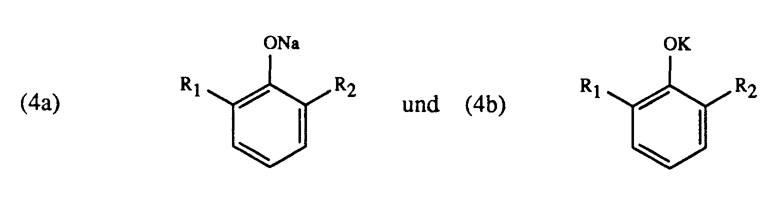
erfolgt, worin R₁ und R₂ die angegebenen Bedeutungen haben.

In den Verbindungen der Formel (1) bedeuten die Substituenten R₁, R₂ und R₃ unabhängig voneinander Alkyl mit 1 bis 4 Kohlenstoffatomen, wie z.B. Methyl, Aethyl, Propyl, Butyl und t-Butyl. R₄ kann die für R₁, R₂ und R₃ angegebenen Bedeutungen haben und zusätzlich auch Wasserstoff bedeuten.

Erfindungsgemäss werden als Basen Gemische aus den Verbindungen der Formeln (4a) und (4b) eingesetzt. Diese Basen erhält man durch Umsetzung der entsprechenden Phenole der Formel (3) mit solchen Basen, die in der Lage sind, die Phenole in Phenolate zu überführen. Beispielsweise können in diesem Zusammenhang Natrium- und Kaliummethanolat, -t-butylat und -amid und vor allem Natrium- und Kaliumhydroxid erwähnt werden. Es sei angemerkt, dass die im erfindungsgemässen Verfahren zu verwendenden Basen noch Spuren dieser starken Basen enthalten können. Auch die Natrium- und Kaliumsalze der Verbindungen der Formel (1) und der Ausgangsverbindung der Formel (3) sind Basen, deren Gegenwart die erfindungsgemässe Umsetzung erleichtert.

In der Regel geschieht die Herstellung des Basengemisches 50, dass eine Verbindung der Formel (3) in einem Rührbehälter in einer Schutzgasatmosphäre aufgeschmolzen wird und man die Schmelze mit einem molaren Unterschuss an Basen in Form einer wässrigen Lösung von z.B. Natrium- und Kaliumhydroxid versetzt. Danach wird unter reduziertem Druck das Wasser aus der Reaktionsmischung entfernt, und man erhält auf diese Weise eine praktisch wasserfreie Suspension der Base in dem Phenol der Formel (3).

Die Reaktion dieser Suspension mit dem Acrylsäurealkylester der Formel (3) kann anschliessend in demselben Rührbehälter erfolgen. Bei Bedarf können auch Teile der Suspension einem anderen Ansatz zur Herstellung der Verbindungen der Formel (1) als Katalysator zugegeben werden.

Vorzugsweise beträgt das Molverhältnis der Verbindung der Formel (4a) zur Verbindung der Formel (4b) in der Reaktionsmischung 0,05:1 bis 20:1. Das Molverhältnis der Verbindungen der Formeln (4a) und (4b) zum Phenol der Formel (3) beträgt mit Vorteil 0,001 bis 0,15. Der Acrylsäurealkylester der Formel (2) wird bevorzugt in bis zu 100%igem molarem Ueberschuss bezogen auf die Verbindung der Formel (3) verwendet. Als besonders geeigneter Temperaturbereich für die Durchführung der Reaktion hat sich das Intervall von 90 bis 150°C erwiesen. Der Druck im Reaktionsgefäss beträgt vorzugsweise 0 bis 4 bar.

Das erfindungsgemässe Verfahren ist besonders geeignet zur Herstellung einer Verbindung der Formel (1), worin R₁ und R₂ t-Butyl sind, R₃ Methyl und R₄ Wasserstoff ist. Dabei wird mit einem 5 bis 40%igen molaren Ueberschuss an Acrylsäuremethylester bezogen auf eingesetztes Phenol der Formel (3) bei einer Temperatur von 100 bis 120°C und unter einem Druck von 0 bis 2 bar gearbeitet. In dem zu verwendenden Basengemisch aus den entsprechenden Verbindungen der Formeln (4a) und (4b) liegen diese Verbindungen im Molverhältnis von 1:0,2 bis 1:10 (Verbindung der Formel (4a) zur Verbindung der Formel (4b)) vor. Als Molverhältnis von Base (Verbindungen der Formeln (4a) und (4b)) zu Phenol der Formel (3) wird 0,005 bis 0,1 gewählt.

Die Verbindungen der Formel (1) lassen sich auf übliche, an sich bekannte Weise isolieren, beispielsweise durch Kristallisation oder Destillation unter reduziertem Druck oder durch Rektifikation, wie z.B. in der schweizerischen Patentanmeldung Nr. 1058/90-0 beschrieben.

In einer bevorzugten Ausführungsform fällt man nach der Reaktion und vor der Isolierung der Verbindung der Formel (1) die Base mit einer Carbonsäure, insbesondere mit Ameisen- oder Essigsäure, aus und filtriert das gebildete Salz ab, wie ebenfalls beispielsweise in der schweizerischen Patentanmeldung Nr. 1058/90-0 beschrieben.

Aufgrund der erheblichen Verkürzung der Reaktionsdauer der erfindungsgemäss durchgeführten Michael-Addition ergeben sich - besonders im Hinblick auf die Durchführung des Verfahrens in industriellem Massstab - deutlich kürzere Kesselbelegungszeiten und somit geringere Periodenkosten.

Das folgende Beispiel erläutert die Erfindung, ohne es darauf zu beschränken. Prozentangaben beziehen sich auf das Gewicht sofern nichts anderes angegeben ist. Dies gilt auch für die übrige Beschreibung und die Patentansprüche.

Beispiel: 600 g geschmolzenes 2,6-Di-tert.-butylphenol werden in einem 2 l-Doppelmantelkolben mit Ankerrührer bei 40°C vorgelegt. Nach Inertisieren mit Stickstoff wird auf eine Temperatur von 100°C aufgeheizt. Man fügt 6,5 g wässrige Kalilauge (50 %) und 2,3 g wässrige Natronlauge (50 %), der Reaktionsmischung hinzu und entwässert bei einer Temperatur von 100°C mit einem Druck von 20 mbar während einer Stunde. Danach wird mit Stickstoff entlastet. Man tropft anschliessend während zwei Stunden 325 g Acrylsäuremethylester bei einer Temperatur von 115°C zu und lässt nachreagieren, bis eine klare Lösung entsteht. Der Druck wird im Reaktionsgefäss langsam auf 10 mbar reduziert, und bei einer Endtemperatur von 115°C entfernt man den Ueberschuss an Acrylsäuremethylester. Nach Entlasten mit Stickstoff neutralisiert man die Base mit 9,8 g Ameisensäure (100 %) und filtriert den gebildeten Niederschlag ab. Die Ausbeute an 3,5-Di-tert.-butyl-4-hydroxyphenyl-1-propionsäuremethylester wird nach einer Gesamtreaktionszeit von 2,5 Stunden in der Reaktionsmasse mittels Flüssigkeitschromatographie auf 93,6 % bestimmt.

## Patentansprüche

1. Verfahren zur Herstellung von Verbindungen der Formel 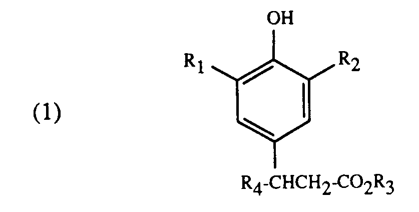 durch Umsetzung von Verbindungen der Formel
(2) R₄-CH=CH-CO₂R₃
mit Verbindungen der Formel 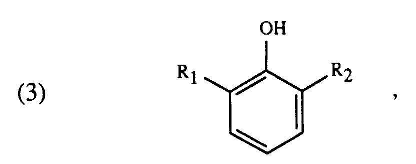 worin R₁ bis R₃ unabhängig voneinander Alkyl mit 1 bis 4 Kohlenstoffatomen sind, und R₄ Wasserstoff oder Alkyl mit 1 bis 4 Kohlenstoffatomen ist, in Gegenwart einer Base und Isolierung der Verbindungen der Formel (I), dadurch gekennzeichnet, dass die Umsetzung einer Verbindung der Formel (2) mit einer Verbindung der Formel (3) in Gegenwart eines Gemisches aus den Verbindungen der Formeln 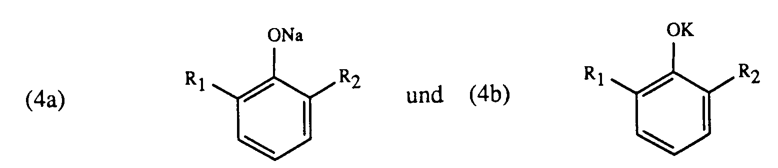 erfolgt, worin R₁ und R₂ die angegebenen Bedeutungen haben.

2. Verfahren nach Anspruch 1, dadurch gekennzeichnet, dass das Molverhältnis der Verbindung der Formel (4a) zur Verbindung der Formel (4b) 0,05:1 bis 20:1 beträgt.

3. Verfahren nach Anspruch 1, dadurch gekennzeichnet, dass das Molverhältnis der Verbindungen der Formeln (4a) und (4b) zur Verbindung der Formel (3) 0,001 bis 0,15 beträgt.

4. Verfahren nach Anspruch 1, dadurch gekennzeichnet, dass die Verbindung der Formel (2) bezogen auf die Verbindung der Formel (3) in bis zu 100%igem molarem Ueberschuss verwendet wird.

5. Verfahren nach Anspruch 1, dadurch gekennzeichnet, dass die Umsetzung der Verbindung der Formel (2) mit der Verbindung der Formel (3) bei einer Temperatur von 90 bis 150°C und unter einem Druck von 0 bis 4 bar ausgeführt wird.

6. Verfahren nach den Ansprüchen 1 bis 5 zur Herstellung einer Verbindung der Formel (1), worin R₁ und R₂ t-Butyl sind, R₃ Methyl und R₄ Wasserstoff ist, dadurch gekennzeichnet, dass die Umsetzung bei einer Temperatur von 100 bis 120°C, unter einem Druck von 0 bis 2 bar und unter Verwendung eines 5 bis 40%igen molaren Ueberschusses an Verbindung der Formel (2) bezogen auf Verbindung der Formel (3), worin R₁ bis R₄ die angegebenen Bedeutungen haben, in Gegenwart eines Germisches aus den Verbindungen der Formeln (4a) und (4b) erfolgt, worin R₁ und R₂ die angegebene Bedeutung haben, die Verbindung der Formel (4a) zur Verbindung der Formel (4b) im Molverhältnis von 1:0,2 bis 1:10 eingesetzt wird und die Verbindungen der Formeln (4a) und (4b) zur Verbindung der Formel (3) im Molverhältnis von 0,005 bis 0,1 eingesetzt werden.

## Claims

1. A process for the preparation of a compound of formula 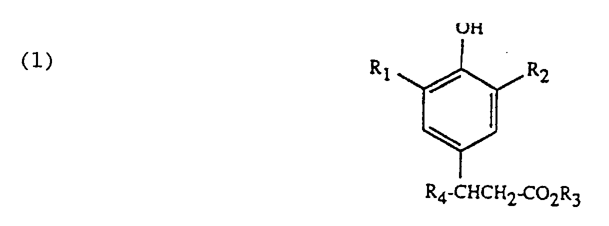 by reacting a compound of formula
(2) R₄-CH=CH-CO₂R₃
with a compound of formula 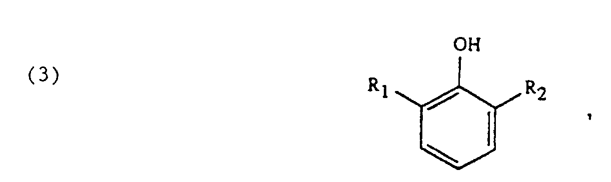 in which R₁ to R₃ are each independently of the other alkyl of 1 to 4 carbon atoms, and R₄ is hydrogen or alkyl of 1 to 4 carbon atoms, in the presence of a base, and isolating said compound of formula (1), which process comprises carrying out the reaction of a compound of formula (2) with a compound of formula (3) in the presence of a mixture of the compounds of formulae 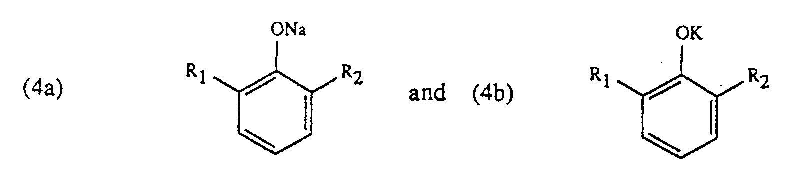 in which R₁ and R₂ have the given meanings.

2. A process according to claim 1, wherein the molar ratio of the compound of formula (4a) to the compound of formula (4b) is 0.05:1 to 20:1.

3. A process according to claim 1, wherein the molar ratio of the compounds of formulae (4a) and (4b) to the compound of formula (3) is 0.001 to 0.15.

4. A process according to claim 1, wherein a molar excess of up to 100 % of the compound of formula (2) is used, based on the compound of formula (3).

5. A process according to claim 1, wherein the reaction of the compound of formula (2) with the compound of formula (3) is carried out at a temperature of from 90 to 150°C and under a pressure of from 0 to 4 bar.

6. A process according to claims 1 to 5 for the preparation of a compound of formula (1) in which R₁ and R₂ are tert-butyl, R₃ is methyl and R₄ is hydrogen, which process comprises carrying out the reaction at a temperature of from 100 to 120°C and under a pressure of from 0 to 2 bar, using a 5 to 40 % molar excess of compound of formula (2), based on compound of formula (3), in which R₁ to R₄ have the given meanings, in the presence of a mixture of the compounds of formulae (4a) and (4b), in which R₁ and R₂ have the given meanings, using the compounds of formulae (4a) and (4b) in the molar ratio of 1:0.2 to 1:10, and the molar ratio of the compounds of formulae (4a) and (4b) to the compound of formula (3) being 0.005 to 0.1.

## Revendications

1. Procédé pour la préparation de composés de formule 1 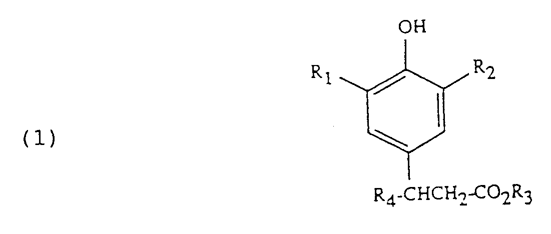 par réaction de composés de formule
(2) R₄-CH=CH-CO₂R₃
avec des composés de formule 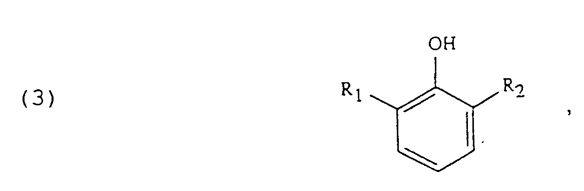 où R₁ à R₃ indépendamment l'un de l'autre représentent un alkyle avec 1 à 4 atomes de carbone, et R₄ représente l'hydrogène ou un alkyle avec 1 à 4 atomes de carbone, en présence d'une base et par isolement des composés de formule (1), caractérisé en ce qu'on met en oeuvre la réaction d'un composé de formule (2) avec un composé de formule (3) en présence d'un mélange de composés de formules 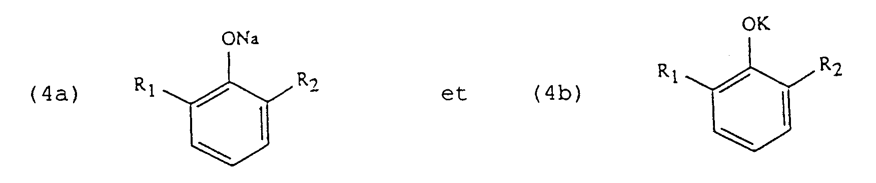 où R₁ et R₂ ont les significations données.

2. Procédé selon la revendication 1, caractérisé en ce que le rapport molaire du composé de formule (4a) au composé de formule (4b) est de 0,05:1 à 20:1.

3. Procédé selon la revendication 1, caractérisé en ce que le rapport molaire des composés de formules (4a) et (4b) au composé de formule (3) est de 0,001 à 0,15.

4. Procédé selon la revendication 1, caractérisé en ce que le composé de formule (2) par rapport au composé de formule (3) est utilisé dans un excès 100% molaires.

5. Procédé selon la revendication 1, caractérisé en ce qu'on effectue la réaction du composé de formule (2) avec le composé de formule (3) à une température de 90 à 150°C et sous une pression de 0 à 4 bars.

6. Procédé selon les revendications 1 à 5 pour la préparation d'un composé de formule (1), où R₁ et R₂ représentent le t-butyle, R₃ représente le méthyle et R₄ l'hydrogène, caractérisé en ce qu'on réalise la réaction à une température de 100 à 120°C, sous une pression de 0 à 2 bars et en utilisant un excès de 5 à 40% molaires en composé de formule (2) par rapport au composé de formule (3), où R₁ à R₄ ont les significations données, en présence d'un mélange de composés de formules (4a) et (4b), où R₁ et R₂ ont les significations données, le composé de formule (4a) par rapport au composé de formule (4b) dans un rapport molaire de 1:0,2 à 1:10 et des composés de formules (4a) et (4b) au composé de formule (3) dans un rapport molaire de 0,005 à 0,1.
